# FASCICULE DE BREVET EUROPEEN

(11) **EP 0 966 207 B1**
(45) Date de publication et mention de la délivrance du brevet: **21.07.2004**
(21) Numéro de dépôt: 98941543.5
(22) Date de dépôt: 05.08.1998
(51) Int. Cl.: A23K 1/16, A61K 9/14, A61K 9/16, C01B 33/193

(54) **COMPOSITION COMPRENANT UN LIQUIDE ABSORBE SUR UN SUPPORT A BASE DE SILICE PRECIPITEE**
ZUSAMMENSTELLUNG BESTEHEND AUS EINER ABSORBIERDEN FLÜSSIGKEIT AUF EINEM TRÄGER AUF BASIS VON FÄLLUNGSKIESELSAÜRE
COMPOSITION COMPRISING A LIQUID ABSORBED ON A SUPPORT BASED ON PRECIPITATE SILICA

(30) Priorité: 06.08.1997 FR 9710250
(43) Date de publication de la demande: 29.12.1999
(73) Titulaire: RHODIA CHIMIE, 92408 Courbevoie Cédex (FR)
(72) Inventeur: VIOT, Jean-François, F-69540 Irigny (FR)
(74) Mandataire: Delenne, Marc
(86) Numéro de dépôt international: PCT/FR1998/001743
(87) Numéro de publication internationale: WO 1999/007237

(56) Documents cités:
- EP-A- 0 345 109
- US-A- 4 617 294
- US-A- 4 717 561
- US-A- 4 775 540

## Description

La présente invention concerne une composition comprenant un liquide, notamment un complément liquide d'alimentation animale, absorbé sur un support à base d'une silice précipitée particulière.

Elle est également relative à l'utilisation de cette silice comme support de liquide

Il est connu de conditionner des liquides, notamment des additifs d'alimentation animale, sur des supports solides, en particulier sur un support silice. Ce conditionnement a généralement pour but de transformer un liquide non ou difficilement manipulable en poudre fluide pouvant être stockée facilement, par exemple en sac, et manipulable plus aisément, et pouvant aussi se disperser sans difficulté et bien se mélanger à d'autres constituants solides divisés.

EP-A-0 345 109 décrit une composition à base d'un liquide absorbée sur une silice précipitée absorbante.

US-A-4 617 294 décrit une composition comprenant la vitamine E absorbé dans un support contenant une silice précipitée avec une taille moyenne de particule de 140 µm à 840 µm, une densité de 224 à 288 kg/m³ et une surface spécifique de 140 à 160 m²/g.

Dans l'exposé qui suit, on entend par composition conditionnée la composition ainsi obtenue, c'est-à-dire un liquide absorbé sur un support silice.

Cette composition conditionnée doit pouvoir être manipulée facilement, ce qui implique une bonne fluidité et un faible poussiérage. Elle doit également présenter une teneur assez importante en matière active (liquide), ainsi qu'une densité élevée Ces différentes exigences sont parfois contradictoires et ne sont pas souvent remplies par les supports silices de l'art antérieur.

Ainsi, l'objet principal de l'invention est de fournir une nouvelle présentation de composition conditionnée possédant de plus, de manière avantageuse, à la fois une bonne fluidité, un poussiérage très faible voire nul et une densité élevée.

La Demanderesse a trouvé que, dans ce but, l'utilisation d'une silice précipitée ayant, entre autres, une morphologie bien spécifique, en l'occurrence une présentation sous forme de billes sensiblement sphériques, et une taille moyenne des particules relativement élevée, en tant que support pour liquides, notamment pour la vitamine E (ou son acétate), était particulièrement satisfaisante.

Dans l'exposé qui suit, la taille moyenne des particules est mesurée selon la norme NF X 11507 (décembre 1970) par tamisage à sec et détermination du diamètre correspondant à un refus cumulé de 50 %.

La densité de remplissage à l'état tassé (DRT) est déterminée selon la norme NF T 30-042.

La prise d'huile DOP est mesurée selon la norme NF T 30-022 (mars 1953) en mettant en oeuvre le dioctylphtalate.

Les volumes poreux donnés sont mesurés par porosimétrie au mercure ; la préparation de chaque échantillon peut se faire comme suit : chaque échantillon est préalablement séché pendant 2 heures en étuve à 200 °C, puis placé dans un récipient à essai dans les 5 minutes suivant sa sortie de l'étuve et dégazé sous vide, par exemple à l'aide d'une pompe à tiroirs rotatifs ; les diamètres de pores sont calculés par la relation de WASHBURN avec un angle de contact théta égal à 140° et une tension superficielle gamma égale à 484 Dynes/cm (porosimètre MICROMERITICS 9300)

La surface spécifique BET est déterminée selon la méthode de BRUNAUER - EMMET - TELLER décrite dans "The journal of the Amencan Chemical Society", Vol. 60, page 309, février 1938 et correspondant à la norme NF T 45007 (novembre 1987)

La surface spécifique CTAB est la surface externe déterminée selon la norme NF T 45007 (novembre 1987) (5.12).

Le temps d'écoulement tₑ des compositions conditionnées, qui illustre leur coulabilité, est mesuré par passage de 50 grammes de produit à travers un silo en verre d'onfice calibré : diamètre du cylindre : 50 mm ; hauteur du cylindre 64 mm ; angle de cône : 53 ° ; diamètre de passage à la base du cône : 8 mm. Selon cette méthode, on remplit le silo, fermé à sa base, à l'aide de 50 grammes de produit ; puis on ouvre sa base et on note après écoulement total desdits 50 grammes le temps de passage, dit temps d'écoulement tₑ, du produit.

L'angle de talus est mesuré selon la norme NF T 20-221.

La composition selon l'invention comprend au moins un liquide absorbé sur un support contenant une silice précipitée, ladite silice précipitée se présentant sous forme de billes sensiblement sphériques et possédant :
- une taille moyenne des billes supérieure à 150 µm,
- une densité de remplissage à l'état tassé (DRT) supéneure à 0,29,
- un taux de refus au tamis ayant une ouverture de mailles de 75 µm d'au moins 92 % en poids.

Ainsi, la silice précipitée utilisée dans la composition conditionnée selon l'invention se présente sous une forme très particulière, en l'occurrence sous forme de billes sensiblement sphériques.

La taille moyenne desdites billes est supérieure à 150 µm, et, avantageusement, égale à au moins 200 µm ; en général, elle est d'au plus 320 µm, de préférence d'au plus 300 µm ; elle peut être comprise entre 200 et 290 µm, en particulier entre 210 et 285 µm, par exemple entre 215 et 280 µm. Cette taille peut notamment être compnse entre 260 et 280 µm.

Ladite silice précipitée présente une densité assez élevée : sa densité de remplissage à l'état tassé (DRT) est supéneure à 0,29. Elle est de préférence d'au moins 0,30, en particulier d'au moins 0,31. Elle peut être d'au moins 0,32.

Cette silice présente un taux de refus au tamis ayant une ouverture de mailles de 75 µm d'au moins 92 % en poids, de préférence d'au moins 93 % en poids. Ceci signifie qu'au moins 92 % en poids, de préférence au moins 93 % en poids, des particules de cette silice sont retenus par un tamis dont l'ouverture de mailles est de 75 µm

Ainsi, cette silice présente une teneur en poids de particules fines faible.

De manière encore plus préférée, son taux de refus au tamis ayant une ouverture de mailles de 75 µm est d'au moins 94 % en poids, notamment d'au moins 95 % en poids ; il peut être par exemple d'au moins 96 % en poids, voire d'au moins 97 % en poids. Il est généralement d'au plus 98 % en poids, en particulier d'au plus 97,5 % en poids.

La silice précipitée utilisée dans la composition conditionnée selon l'invention est donc peu poussiérante.

Elle présente une prise d'huile (DOP) d'au moins 250 ml/100g, de préférence comprise entre 250 et 280 ml/100g. Celle-ci peut être comprise entre 255 et 275 ml/100g, par exemple entre 255 et 270 ml/100g

Ladite silice possède habituellement un volume poreux (V_{d1}) constitue par les pores de diamètre inférieur à 1 µm inféneur à 1,95 cm³/g, en particulier inféneur à 1,90 cm³/g.

Sa surface spécifique BET est généralement comprise entre 140 et 240 m²/g, notamment entre 140 et 200 m²/g. Elle est par exemple comprise entre 150 et 190 m²/g Elle peut être en particulier comprise entre 160 et 170 m²/g.

Sa surface spécifique CTAB peut être compnse entre 140 et 230 m²/g, notamment entre 140 et 190 m²/g. Elle est par exemple comprise entre 150 et 180 m²/g, en particulier entre 150 et 165 m²/g.

Elle présente en général une humidité réduite ; son taux d'humidité (perte au séchage à 105 °C pendant 2 heures) est de préférence inféneur à 5 % en poids.

De manière avantageuse, la silice employée dans la composition selon l'invention est issue du séchage au moyen d'un atomiseur à buses d'une suspension de silice obtenue par précipitation. De préférence, ladite suspension de silice à sécher présente un taux de matière sèche compris entre 22 et 24 % en poids, en particulier entre 22,5 et 23,5 % en poids.

Cette silice peut être préparée par un procédé du type comprenant la réaction d'un silicate avec un agent acidifiant ce par quoi l'on obtient une suspension de silice précipitée, puis la séparation et le séchage à l'aide d'un atomiseur à buses de cette suspension, la précipitation étant réalisée de la manière savante :
(1) on forme un pied de cuve initial comportant au moins une partie de la quantité totale de silicate engagé dans la réaction et, en général, au moins un électrolyte, la concentration en silicate (exprimée en SiO₂) dans ledit pied de cuve initial étant inférieure à 100 g/l, notamment à 90 g/l, et la concentration en électrolyte (sulfate de sodium par exemple) dans ledit pied de cuve initial étant inférieure à 17 g/l, par exemple inféneure à 14 g/l,
(2) on ajoute l'agent acidifiant audit pied de cuve jusqu'à l'obtention d'une valeur du pH du milieu réactionnel d'au moins environ 7, généralement compnse entre environ 7 et 8,
(3) on ajoute au milieu réactionnel de l'agent acifiant et, le cas échéant, simultanément la quantité restante du silicate,
la suspension à sécher présentant un taux de matière sèche compris entre 22 et 24 % en poids, en particulier entre 22,5 et 23,5 % en poids.

Il est à noter, d'une manière générale, que le procédé concerné est un procédé de synthèse de silice de précipitation, c'est-à-dire que l'on fait agir, dans des conditions particulières, un agent acidifiant sur un silicate.

Le choix de l'agent acidifiant et du silicate se fait d'une manière bien connue en soi.

On peut rappeler que l'on utilise généralement comme agent acidifiant un acide minéral fort tel que l'acide sulfurique, l'acide nitrique ou l'acide chlorhydrique, ou un acide organique tel que l'acide acétique, l'acide formique ou l'acide carbonique.

L'agent acidifiant peut être dilué ou concentré ; sa normalité peut être comprise entre 0,4 et 8 N, par exemple entre 0,6 et 1,5 N.

En particulier, dans le cas où l'agent acidifiant est l'acide sulfurique, sa concentration peut être comprise entre 40 et 180 g/l, par exemple entre 60 et 130 g/l.

On peut par ailleurs utiliser en tant que silicate toute forme courante de silicates tels que métasilicates, disilicates et avantageusement un silicate de métal alcalin, notamment le silicate de sodium ou de potassium.

Le silicate peut présenter une concentration exprimée en silice comprise entre 40 et 330 g/l, en particulier entre 60 et 300 g/l, par exemple entre 60 et 250 g/l

De manière générale, on emploie, comme agent acidifiant, l'acide sulfurique, et, comme silicate, le silicate de sodium.

Dans le cas où l'on utilise le silicate de sodium, celui-ci présente, en général, un rapport pondéral SiO₂/Na₂O compris entre 2 et 4, par exemple entre 3,0 et 3,7

Le pied de cuve initial comprend, en général, un électrolyte. Le terme d'électrolyte s'entend ici dans son acceptation normale, c'est-à-dire qu'il signifie toute substance ionique ou moléculaire qui, lorsqu'elle est en solution, se décompose ou se dissocie pour former des ions ou des particules chargées. On peut citer comme électrolyte un sel du groupe des sels des métaux alcalins et alcalino-terreux, notamment le sel du métal de silicate de départ et de l'agent acidifiant, par exemple le chlorure de sodium dans le cas de la réaction d'un silicate de sodium avec l'acide chlorhydrique ou, de préférence, le sulfate de sodium dans le cas de la réaction d'un silicate de sodium avec l'acide sulfurique.

Dans le cas (préféré) d'un pied de cuve de départ ne comprenant qu'une partie de la quantité totale de silicate engagé dans la réaction, on procède, dans l'étape (3), à une addition simultanée d'agent acidifiant et de la quantité restante de silicate

Cette addition simultanée est de préférence réalisée de manière telle que la valeur du pH soit constamment égale (à +/- 0,2 près) à celle atteinte à l'issue de l'étape (2).

En général, dans une étape suivante, on ajoute au milieu réactionnel une quantité supplémentaire d'agent acidifiant, de préférence jusqu'à l'obtention d'une valeur du pH du milieu réactionnel comprise entre 3 et 6,5, en particulier entre 4 et 6,5.

Il peut être alors avantageux d'effectuer, après cette addition d'une quantité supplémentaire d'agent acidifiant, un mûrissement du milieu réactionnel, ce mûnssement pouvant par exemple durer de 2 à 60 minutes, notamment de 3 à 20 minutes.

Dans le cas d'un pied de cuve de départ comprenant la quantité totale du silicate engagé dans la réaction, on procède, dans l'étape (3), à une addition d'agent acidifiant, de préférence jusqu'à l'obtention d'une valeur du pH du milieu réactionnel compnse entre 3 et 6,5, en particulier entre 4 et 6,5.

Il peut être également alors avantageux d'effectuer, après cette étape (3), un mûrissement du milieu réactionnel, ce mûrissement pouvant par exemple durer de 2 à 60 minutes, notamment de 3 à 20 minutes.

La température du milieu réactionnel est généralement comprise entre 70 et 98 °C.

Selon une vanante du procédé, la réaction est effectuée à une température constante, de préférence comprise entre 80 et 95 °C.

Selon une autre vanante (préférée) du procédé, la température de fin de réaction est plus élevée que la température de début de réaction : ainsi, on maintient la température au début de la réaction de préférence entre 70 et 95 °C, puis on augmente la température, de préférence jusqu'à une valeur comprise entre 80 et 98 °C, valeur à laquelle elle est maintenue jusqu'à la fin de la réaction.

On obtient, à l'issue des étapes qui viennent d'être décrites, une bouillie de silice qui est ensuite séparée (séparation liquide-solide).

En général, ladite séparation comprend une filtration et un lavage à l'aide d'un filtre équipé d'un moyen de compactage.

Ce filtre peut être un filtre à bande équipé d'un rouleau assurant le compactage

Néanmoins, de préférence, ce filtre est un filtre presse, la séparation comprend alors en général une filtration, un lavage puis un compactage, au moyen dudit filtre

La suspension de silice précipitée ainsi récupérée (gâteau de filtration) est ensuite séchée par atomrsation, au moyen d'un atomiseur à buses

De manière très préférée, cette suspension doit présenter immédiatement avant son séchage un taux de matière sèche compris entre 22 et 24 % en poids, en particulier entre 22,5 et 23,5 % en poids.

Il y a lieu de noter que le gâteau de filtration n'est pas toujours dans des conditions permettant une atomisation notamment à cause de sa viscosité élevée. D'une manière connue en soi, on soumet alors le gâteau à une opération de délitage. Cette opération peut être réalisée par passage du gâteau dans un broyeur de type colloïdal ou à bille. Le délitage est généralement effectué en présence d'un composé de l'aluminium, en particulier d'aluminate de sodium. L'opération de délitage permet notamment d'abaisser la viscosité de la suspension à sécher ultérieurement.

La Demanderesse a découvert que la silice précipitée définie plus haut, donc ayant une morphologie bien spécifique, en l'occurrence une présentation sous forme de billes sensiblement sphériques, et denses, une taille moyenne des particules relativement élevée, présentait une bonne fluidité et un caractère peu poussiérant, et convenait particulièrement bien au conditionnement des liquides

A titre de liquides, on peut notamment citer les liquides organiques tels que les acides organiques, les agents tensio-actifs, par exemple utilisés en détergence, du type anionique tels que les sulfonates ou du type non ionique tels que les alcools, les additifs organiques pour caoutchouc, les pesticides. On peut utiliser à titre de liquides des agents conservateurs (acide phosphorique, acide propronique notamment), des arômes, des colorants.

La Demanderesse a constaté que la silice précipitée décnte précédemment était particulièrement adaptée au conditionnement des compléments liquides d'alimentation, notamment d'alimentation animale.

Ainsi, le liquide contenu dans la composition conditionnée conforme à l'invention est de préférence un complément liquide d'alimentation animale. On peut notamment citer la choline, le chlorhydrate de choline, les vitamines telles que les vitamines A, B, C, D, K et, de préférence, la vitamine E (ou son acétate).

L'opération d'absorption du liquide sur le support à base de ladite silice précipitée peut s'effectuer de manière classique, en particulier par pulvérisation du liquide sur la silice dans un mélangeur.

Si la quantité de liquide absorbé dépend en général de l'application recherchée, la composition selon l'invention présente habituellement, notamment dans le cas de la vitamine E (ou de son acétate), une teneur en liquide d'au moins 50 % en poids, en particulier compnse entre 50 et 65 % en poids, notamment entre 50 et 60 % en poids (par rapport au poids total de la composition) ; elle peut être par exemple compnse entre 52 et 56 % en poids.

Des teneurs en liquide encore supérieures peuvent être utilisées, notamment dans le cas du chlorhydrate de choline.

La composition conditionnée selon l'invention, du fait de la présence de la silice précipitée ayant les caractéristiques sus-mentionnées, présente, de manière avantageuse, un poussiérage très faible voire nul et une excellente fluidité, combinés à une densité élevée.

Ainsi, de manière préférée, ladite silice précipitée confère à cette composition, en particulier dans le cas de la vitamine E (ou de son acétate), un taux de refus au tamis ayant une ouverture de mailles de 75 µm d'au moins 97 % en poids, notamment d'au moins 98 % en poids. Ce taux, en particulier dans le cas de la vitamine E (ou de son acétate), est préférentiellement d'au moins 99 % en poids, par exemple d'au moins 99,5 % en poids, voire d'au moins 99,7 % en poids ; il peut même être d'au moins 99,8 % en poids. Cette composition présente ainsi une teneur en poids de particules fines extrêmement faible. Elle est donc très peu ou même pas du tout poussiérante.

De plus, la composition conditionnée selon l'invention, en particulier dans le cas de la vitamine E (ou de son acétate), présente, de manière préférée, un temps d'écoulement tₑ (mesuré pour 50 grammes de produit et pour un diamètre de passage de 8 mm) inférieur à 10 secondes, notamment inférieur à 9 secondes et, par exemple, d'au plus 8 secondes, ce qui témoigne de son excellente fluidité.

Il peut être noté que l'angle de talus de cette composition, notamment dans le cas de la vitamine E (ou de son acétate), est généralement d'au plus 31 °, et peut être inférieur à 30°.

La composition conditionnée conforme à l'invention, en particulier dans le cas de la vitamine E (ou de son acétate), présente habituellement une densité de remplissage à l'état tassé (DRT) d'au moins 0,63, par exemple d'au moins 0,65, et peut être d'au moins 0,70.

L'invention a également pour objet l'utilisation de la silice précipitée décnte précédemment comme support de liquide, tel que par exemple l'un des liquides cités plus haut.

L'exemple suivant illustre l'invention sans toutefois en limiter la portée.

### EXEMPLE

1) Dans un réacteur en acier inoxydable muni d'un système d'agitation par hélices et d'un chauffage par double enveloppe, on introduit :
   - 345 litres d'eau
   - 7,5 kg de Na₂SO₄
   - 586 litres de silicate de sodium aqueux présentant un rapport pondéral SiO₂/Na₂O égal à 3,5 et une densité à 20 °C égale à 1,133

   La concentration en silicate exprimée en SiO₂ dans le pied de cuve initial est ainsi de 85 g/l. Le mélange est alors porté à une température de 79 °C tout en le maintenant sous agitation. On y introduit ensuite 386 litres d'acide sulfurique dilué, de densité à 20 °C égale à 1,050, jusqu'à obtenir dans le mileu réactionnel une valeur de pH (mesurée à sa température) égale à 8,0. La température de la réaction est de 79 °C pendant les 25 premières minutes ; elle est ensuite portée de 79 à 86 °C en 15 minutes, puis maintenue à 86 ° C jusqu'à la fin de la réaction.
   On introduit ensuite (c'est-à-dire lorsque le pH du milieu réactionnel a atteint la valeur de 8,0) conjointement dans le milieu réactionnel 83 litres de silicate de sodium aqueux du type décrit ci-avant et 134 litres d'acide sulfurique, également du type décnt ci-avant, cette introduction simultanée d'acide et de silicate étant réalisée de manière telle que le pH du milieu réactionnel, pendant la période d'introduction, soit constamment égal à 8,0 ± 0,1. Après introduction de la totalité du silicate, on continue à introduire de l'acide dilué pendant 9 minutes de manière à amener le pH du milieu réactionnel à une valeur égale à 5,2. Après cette introduction d'acide, on maintient la bouillie réactionnelle obtenue pendant 5 minutes sous agitation.
   La durée totale de la réaction est de 119 minutes.
   On obtient ainsi une bouillie ou suspension de silice précipitée qui est ensuite filtrée et lavée au moyen d'un filtre presse à plateaux verticaux (lesdits plateaux étant équipés de membrane déformable permettant de compnmer le gâteau de filtration par introduction d'air sous pression), à une pression de 7,5 bars et pendant le temps nécessaire afin d'obtenir un gâteau de silice dont la perte au feu est égale à 77,1 % (donc un taux de matière sèche de 22,9 % en poids).
   Le gâteau obtenu est ensuite fluidifié par action mécanique et chimique (ajout d'une quantité d'aluminate de sodium correspondant à un rapport pondéral Al/SiO₂ de 3000 ppm). Après cette opération de délitage, la bouillie résultante, de pH égal à 6,6, est séchée au moyen d'un atomiseur à buses.
   La silice précipitée obtenue P1 se présente sous forme de billes sensiblement sphériques et possède les caractéristiques supplémentaires suivantes :
   - taille moyenne des particules 270 µm
   - DRT 0,31
   - taux de refus au tamis 75 µm 96,7 %
   - prise d'huile DOP 265 ml/100g
   - volume poreux (V_{d1}) constitué
      par les pores de d < 1 µm < 1,90 cm³/g
   - surface spécifique BET 165 m²/g
   - surface spécifique CTAB 156 m²/g
2) On met de la vitamine E sur un support formé par la silice P1 préparée en 1)
   La mise sur support de la vitamine E s'effectue dans un mélangeur en V de 7 litres tournant à 20 tours/min, avec un axe intérieur tournant à 1900 tours/min, muni de plaques au travers desquelles est pulvérisée la vitamine E et sur lesquelles sont fixés des couteaux émotteurs.
   On charge la totalité de la silice P1 dans le mélangeur, puis on pulvénse la vitamine E (à une température de 70 °C et à un débit de 100 ml/min) sur cette silice. On mélange pendant 15 minutes, puis on homogénéise durant 2 minutes supplémentaires
   La composition conditionnée alors obtenue contient 45 % en poids de silice précipitée P1 et 55 % en poids de vitamine E et possède les caractéristiques supplémentaires suivantes :
   - taux de refus au tamis 75 µm 99,7 %
   - temps d'écoulement tₑ 8 secondes
   - angle de talus 29,9°
   - DRT > 0,65

Ainsi, cette composition conditionnée à base d'un support silice précipitée, sous forme de billes sensiblement sphériques, présente une très bonne fluidité (ce qui est illustré notamment par un faible temps d'écoulement tₑ) et n'est pas du tout poussiérante (ce qui est illustré en particulier par un taux de refus au tamis ayant une ouverture de mailles de 75 µm très élevé), tout en ayant une forte densité.

## Revendications

1. Composition conditionnée comprenant au moins un liquide absorbé sur un support contenant une silice précipitée, **caractérisée en ce que** ladite silice se présente sous forme de billes sensiblement sphériques et possède :
- une taille moyenne des billes supérieure à 150 µm,
- une densité de remplissage à l'état tassé (DRT) supérieure à 0,29,
- un taux de refus au tamis ayant une ouverture de mailles de 75 µm d'au moins 92 % en poids,
- une prise d'huile DOP d'au moins 250 ml/100g.

2. Composition selon la revendication 1, **caractérisée en ce que** la dite silice possède une taille moyenne des billes d'au moins 200 µm, en particulier comprise entre 200 et 290 µm.

3. Composition selon l'une des revendications 1 et 2, **caractérisée en ce que** ladite silice possède une densité de remplissage à l'état tassé d'au moins 0,30, en particulier d'au moins 0,31.

4. Composition selon l'une des revendications 1 à 3, **caractérisée en ce que** ladite silice possède un taux de refus au tamis ayant une ouverture de mailles de 75 µm d'au moins 93 % en poids, en particulier d'au moins 94 % en poids.

5. Composition selon l'une des revendications 1 à 4, **caractérisée en ce que** ladite silice possède une prise d'huile DOP comprise entre 250 et 280 ml/100g, en particulier entre 255 et 275 ml/100g.

6. Composition selon l'une des revendications 1 à 5, **caractérisée en ce que** la dite silice possède un volume poreux (V_{d1}) constitué par les pores de diamètre inférieur à 1 µm inférieur à 1,95 cm³/g, en particulier inférieur à 1,90 cm³/g.

7. Composition selon l'une des revendications 1 à 6, **caractérisée en ce que** ladite silice est issue du séchage au moyen d'un atomiseur à buses d'une suspension de silice obtenue par précipitation.

8. Composition selon la revendication 7, **caractérisée en ce que** ladite suspension de silice présente, avant séchage, un taux de matière sèche compris entre 22 et 24 % en poids, de préférence entre 22,5 et 23,5 % en poids.

9. Composition selon l'une des revendications 1 à 8, **caractérisée en ce que** ladite composition présente une teneur en liquide d'au moins 50 % en poids, en particulier comprise entre 50 et 65 % en poids.

10. Composition selon l'une des revendications 1 à 9, **caractérisée en ce que** ledit liquide est un agent conservateur, un arôme, un colorant, un complément liquide d'alimentation animale.

11. Composition selon l'une des revendications 1 à 10, **caractérisée en ce que** ledit liquide est la vitamine E ou l'acétate de vitamine E.

12. Composition selon l'une des revendications 1 à 11, **caractérisée en ce que** ladite composition possède un taux de refus au tamis ayant une ouverture de mailles de 75 µm d'au moins 97 % en poids, en particulier d'au moins 98 % en poids.

13. Composition selon l'une des revendications 1 à 12, **caractérisée en ce que** ladite composition présente un temps d'écoulement tₑ, pour 50 grammes et pour un diamètre de passage de 8 mm, inférieur à 10 secondes, de préférence inférieur à 9 secondes.

14. Utilisation d'une silice précipitée comme support de liquide, notamment de complément liquide d'alimentation animale, **caractérisée en ce que** ladite silice se présente sous forme de billes sensiblement sphériques et possède :
- une taille moyenne des billes supérieure à 150 µm, de préférence supérieure à 200 µm,
- une densité de remplissage à l'état tassé (DRT) supérieure à 0,29, de préférence d'au moins 0,30,
- un taux de refus au tamis ayant une ouverture de mailles de 75 µm d'au moins 92 % en poids, de préférence d'au moins 93 % en poids,
- une prise d'huile DOP d'au moins 250 ml/100 g.

15. Utlisation selon la revendication 14, **caractérisée en ce que** ladite silice possède une prise d'huile DOP comprise entre 250 et 280 ml/100 g.

16. Utilisation selon l'une des revendications 14 et 15, **caractérisée en ce que** la dite silice possède un volume poreux (V_{d1}) constitué par les pores de diamètre inférieur à 1 µm inférieur à 1,95 cm³/g, en particulier inférieur à 1,90 cm³/g.

17. Utilisation selon l'une des revendications 14 à 16, **caractérisée en ce que** ladite silice est issue du séchage au moyen d'un atomiseur à buses d'une suspension de silice obtenue par précipitation, ladite suspension de silice présentant de préférence, avant séchage, un taux de matière sèche compris entre 22 et 24 % en poids.

18. Utilisation selon l'une des revendications 14 à 17, **caractérisée en ce que** ledit liquide est la vitamine E ou l'acétate de vitamine E.

## Patentansprüche

1. Konditionierte Zusammensetzung umfassend wenigstens eine Flüssigkeit, die auf einem Träger absorbiert ist, der eine ausgefällte Silica enthält, **dadurch gekennzeichnet, dass** die Silica in Form von ungefähr kugelförmigen Kugeln vorliegt und folgendes besitzt:
- eine mittlere Größe der Kugeln von größer als 150 µm,
- eine Fülldichte in dichtgedrängtem Zustand (DRT) von größer als 0,29,
- eine Rückstandsrate in einem Sieb mit einer Öffnung der Maschen von 75 µm von wenigstens 92 Gew.-%,
- eine Ölzahl DOP von wenigstens 250 ml/100 g.

2. Die Zusammensetzung gemäß Anspruch 1, **dadurch gekennzeichnet, dass** die Silica eine mittlere Größe der Kugeln von wenigstens 200 µm besitzt, insbesondere zwischen 200 und 290 µm.

3. Die Zusammensetzung gemäß einem der Ansprüche 1 und 2, **dadurch gekennzeichnet, dass** die Silica eine Fülldichte in dichtgedrängtem Zustand von wenigstens 0,30, insbesondere wenigstens 0,31, aufweist.

4. Die Zusammensetzung gemäß einem der Ansprüche 1 bis 3, **dadurch gekennzeichnet, dass** die Silica eine Rückstandsrate in einem Sieb mit Öffnungen der Maschen von 75 µm von wenigstens 93 Gew.-% besitzt, insbesondere wenigstens 94 Gew.-%.

5. Die Zusammensetzung gemäß einem der Ansprüche 1 bis 4, **dadurch gekennzeichnet, dass** die Silica eine Ölzahl DOP zwischen 250 und 280 ml/100 g besitzt, insbesondere zwischen 255 und 275 ml/100 g.

6. Die Zusammensetzung gemäß einem der Ansprüche 1 bis 5, **dadurch gekennzeichnet, dass** die Silica ein Porenvolumen (V_{d1}), das durch die Poren mit einem Durchmesser von weniger als 1 µm gebildet ist, von weniger als 1,95 cm³/g besitzt, insbesondere von weniger als 1,90 cm³/g.

7. Die Zusammensetzung gemäß einem der Ansprüche 1 bis 6, **dadurch gekennzeichnet, dass** die Silica aus der Trocknung einer Suspension von Silica, die durch Ausfällung erhalten worden ist, mittels einem Düsenzerstäuber stammt.

8. Die Zusammensetzung gemäß Anspruch 7, **dadurch gekennzeichnet, dass** die Suspension von Silica vor der Trocknung einen Trockensubstanzgehalt zwischen 22 und 24 Gew.-%, bevorzugt zwischen 22,5 und 23,5 Gew.-%, besitzt.

9. Die Zusammensetzung gemäß einem der Ansprüche 1 bis 8, **dadurch gekennzeichnet, dass** die Zusammensetzung einen Flüssigkeitsgehalt von wenigstens 50 Gew.-% aufweist, insbesondere zwischen 50 und 65 Gew.-%.

10. Die Zusammensetzung gemäß einem der Ansprüche 1 bis 9, **dadurch gekennzeichnet, dass** die Flüssigkeit ein Konservierungsmittel, ein Aroma, ein Farbstoff, ein flüssiges Nahrungsergänzungsmittel für Tiere ist.

11. Die Zusammensetzung gemäß einem der Ansprüche 1 bis 10, **dadurch gekennzeichnet, dass** die Flüssigkeit Vitamin E oder das Acetat von Vitamin E ist.

12. Die Zusammensetzung gemäß einem der Ansprüche 1 bis 11, **dadurch gekennzeichnet, dass** die Zusammensetzung eine Rückstandsrate in einem Sieb mit Öffnungen der Maschen von 75 µm von wenigstens 97 Gew.-%, insbesondere von wenigstens 98 Gew.-%, besitzt.

13. Die Zusammensetzung gemäß einem der Ansprüche 1 bis 12, **dadurch gekennzeichnet, dass** die Zusammensetzung eine Auslaufzeit t_{θ}, für 50 g und für einen Durchgangsdurchmesser von 8 mm, von weniger als 10 Sekunden, bevorzugt weniger als 9 Sekunden besitzt.

14. Eine Verwendung einer ausgefällten Silica als Träger von Flüssigkeit, insbesondere von flüssigem Nahrungsergänzungsmittel für Tiere, **dadurch gekennzeichnet, dass** die Silica in Form von ungefähr kugelförmigen Kugeln vorliegt und folgendes besitzt:
- eine mittlere Größe der Kugeln von größer als 150 µm, bevorzugt größer als 200 µm,
- eine Fülldichte in dichtgedrängtem Zustand (DRT) von größer als 0,29, bevorzugt von wenigstens 0,30,
- eine Rückstandsrate in einem Sieb mit Öffnungen der Maschen von 75 µm von wenigstens 92 Gew.-%, bevorzugt wenigstens 93 Gew.-%,
- eine Ölzahl DOP von wenigstens 250 ml/100 g.

15. Die Verwendung gemäß Anspruch 14, **dadurch gekennzeichnet, dass** die Silica eine Ölzahl DOP zwischen 250 und 280 ml/100 g besitzt.

16. Die Verwendung gemäß einem der Ansprüche 14 und 15, **dadurch gekennzeichnet, dass** die Silica ein Porenvolumen (V_{d1}), das durch die Poren mit einem Durchmesser von weniger als 1 µm gebildet ist, von weniger als 1,95 cm³/g besitzt, insbesondere von weniger als 1,90 cm³/g.

17. Die Verwendung gemäß einem der Ansprüche 14 bis 16, **dadurch gekennzeichnet, dass** die Silica aus der Trocknung einer Suspension von Silica, die durch Ausfällung erhalten worden ist, mittels einem Düsenzerstäuber stammt, wobei die Suspension von Silica bevorzugt vor der Trocknung einen Trockensubstanzgehalt zwischen 22 und 24 Gew.-% aufweist.

18. Die Verwendung gemäß einem der Ansprüche 14 bis 17, **dadurch gekennzeichnet, dass** die Flüssigkeit Vitamin E oder das Acetat von Vitamin E ist.

## Claims

1. Conditioned composition comprising at least one liquid absorbed on a support containing a precipitated silica, **characterized in that** the said silica is in the form of substantially spherical beads and has:
- an average bead size in excess of 150 µm,
- a packed filling density (PFD) in excess of 0.29,
- a 75 µm screen oversize of at least 92% by weight,
- a DOP oil absorption value of at least 250 ml/100 g.

2. Composition according to Claim 1, **characterized in that** the said silica has an average bead size of at least 200 µm, in particular between 200 and 290 µm.

3. Composition according to one of Claims 1 and 2, **characterized in that** the said silica has a packed filling density of at least 0.30, in particular of at least 0.31.

4. Composition according to one of Claims 1 to 3, **characterized in that** the said silica has a 75 µm screen oversize of at least 93% by weight, in particular of at least 94% by weight.

5. Composition according to one of Claims 1 to 4, **characterized in that** the said silica has a DOP oil absorption value of between 250 and 230 ml/100 g, in particular between 255 and 275 ml/100 g.

6. Composition according to one of Claims 1 to 5, **characterized in that** the said silica has a pore volume (V_{d1}) made up of pores having a diameter smaller than 1 µm of less than 1.95 cm³/g, in particular of less than 1.90 cm³/g.

7. Composition according to one of Claims 1 to 6, **characterized in that** the said silica results from the use of a nozzle atomizer to dry a suspension of silica obtained by precipitation.

8. Composition according to Claim 7, **characterized in that**, before it is dried, the said silica suspension has a solids content of between 22 and 24% by weight, preferably between 22.5 and 23.5% by weight.

9. Composition according to one of Claims 1 to 8, **characterized in that** the said composition has a liquid content of at least 50% by weight, in particular between 50 and 65% by weight.

10. Composition according to one of Claims 1 to 9, **characterized in that** the said liquid is a preserving agent, a flavour, a colorant or a liquid supplement for animal feed.

11. Composition according to one of Claims 1 to 10, **characterized in that** the said liquid is vitamin E or vitamin E acetate.

12. Composition according to one of Claims 1 to 11, **characterized in that** the said composition has a 75 µm screen oversize of at least 97% by weight, in particular of at least 98% by weight.

13. Composition according to one of Claims 1 to 12, **characterized in that** the said composition has a flow time t_{f}, for 50 grams and for a passage diameter of 8 mm, of less than 10 seconds, preferably of less than 9 seconds.

14. Use of a precipitated silica as a liquid support, in particular as a liquid supplement for animal feed, **characterized in that** the said silica is in the form of substantially spherical beads and has:
- an average bead size in excess of 150 µm, preferably in excess of 200 µm,
- a packed filling density (PFD) in excess of 0.29, preferably of at least 0.30,
- a 75 µm screen oversize of at least 92% by weight, preferably of at least 93% by weight,
- a DOP oil absorption valve of at least 250 ml/100 g.

15. Use according to Claim 14, **characterized in that** the said silica has a DOP oil absorption value of between 250 and 280 ml/100 g.

16. Use according to one of Claims 14 and 15, **characterized in that** the said silica has a pore volume (V_{d1}) made up of pores having a diameter smaller than 1 µm of less than 1.95 cm³/g, in particular of less than 1.90 cm³/g.

17. Use according to one of Claims 14 to 16, **characterized in that** the said silica results from the use of a nozzle atomizer to dry a suspension of silica obtained by precipitation, the said silica suspension preferably having a solids content of between 22 and 24% by weight before it is dried.

18. Use according to one of Claims 14 to 17, **characterized in that** the said liquid is vitamin E or vitamin E acetate.
